Europäisches Patentamt

European Patent Office

Office européen des brevets

(11) Publication number: **0 234 046 B1**

# EUROPEAN PATENT SPECIFICATION

(45) Date of publication of patent specification: 20.03.91    (51) Int. Cl.⁵: **A61M 1/10**

(21) Application number: 86118095.8

(22) Date of filing: 29.12.86

(54) Intra-aortic balloon apparatus.

(30) Priority: 31.12.85 US 815030

(43) Date of publication of application:
02.09.87 Bulletin 87/36

(45) Publication of the grant of the patent:
20.03.91 Bulletin 91/12

(84) Designated Contracting States:
DE FR GB

(56) References cited:
EP-A- 0 192 574          GB-A- 1 566 674
US-A- 3 585 983          US-A- 4 024 873
US-A- 4 077 394          US-A- 4 274 423
US-A- 4 362 150          US-A- 4 456 000

(73) Proprietor: **AISIN SEIKI KABUSHIKI KAISHA
1, Asahi-machi 2-Chome
Kariya City Aichi Pref.(JP)**

(72) Inventor: **Kantrowitz, Adrian
70, Gallogly Road
Pontiac MI 48055(US)**
Inventor: **Freed, Paul S.
1486, Sodon Court
Bloomfield Hills MI 48013(US)**
Inventor: **Bar-Lev, Avi
27600, East Gateway
Farmington Hills MI 48018(US)**
Inventor: **Mushika, Sadahiko
2-5, Yushima 2-chome Bunkyo-ku
Tokyo(JP)**
Inventor: **Suzuki, Akira
100, Iyama Minaminakane-cho
Nishio-shi Aichi-ken(JP)**

(74) Representative: **Grams, Klaus Dieter, Dipl.-Ing.
et al
Patentanwaltsbüro Tiedtke-Bühling-Kinne-
Grupe-Pellmann-Grams-Struif Winter-Roth
Bavariaring 4
W-8000 München 2(DE)**

## Description

This invention relates to an intra-aortic balloon apparatus according to the pre-characterizing portion of claim 1.

An intra-aortic balloon pump apparatus basically comprises a catheter through which a fluid such as helium gas is passed, and a balloon attached to the distal end of the catheter which is cyclically inflated by feeding helium gas into the balloon from an external source via the catheter. The apparatus is inserted into the patient, usually through the femoral artery, either percutaneously or through an incision, and advanced until it lies within the thoracic aorta. There it is made to inflate and deflate synchronously with the heart so as to reduce the load on the left ventricle and increase the amount of coronary blood flow.

An example of an intra-aortic balloon pump apparatus capable of being inserted into a living body by percutaneous introduction (Seldinger technique) is disclosed in U S -A-4,362,150.

During use of an intra-aortic balloon pump apparatus, an electro-cardiogram and an arterial pressure signal are utilized to determine the time of inflation and deflation of the balloon. An example or such an apparatus is disclosed in the specification of U S -A-3,585,983. Here a pressure transducer is disposed at the distal end of the balloon and a signal produced by the pressure transducer is led out from the patient's body through the catheter. This conventional arrangement has the limitation that the balloon apparatus cannot be introduced into the patient percutaneously and, hence, the apparatus is limited to inhospital use.

A different arrangement is set forth in the specification or U S-A-4,077,394. Here a small tube leads from the tip of the balloon through the catheter, feeding aortic pressure to a sensor located externally to the patient. However, this apparatus possesses the same drawback mentioned above, namely the fact that percutaneous introduction is not feasible.

If the method of measuring aortic pressure described in U S-A-4,077,394 were to utilize the central tube mentioned in U S-A-4,362,150, then the apparatus could be applied in the same manner. However, the system still would be prone to considerable external noise and distortion of the pressure signal which would make computerized recognition of the pressure signal unreliable.

Accordingly, an object of the present invention is to enable the accurate measurement of central aortic pressure by an intra-aortic balloon apparatus which is also capable of being introduced percutaneously.

According to the present invention, the foregoing objects are attained by providing an intra-aortic balloon apparatus having the features of claim 1.

US-A-4 274 423 discloses a catheter comprising some of the features of the second catheter of the present invention. This known catheter, however, is not adapted to be used within another catheter device. A design, that would enable a measuring device provided at the distal end of the known catheter to measure a parameter free of any disturbances caused by a further, surrounding catheter, can not be gathered from US-A-4 274 423.

In an embodiment of the present invention, the second catheter of the intra aortic balloon pump apparatus comprises a stainless steel tube and leads from the pressure transducer are extended to the second connector by being passed through, the interior of the second catheter. In another embodiment, the second connector is provided with a medical fluid port communicating with the aorta through a passageway defined between the central tubular member and the second catheter.

Advantageous modifications of the invention derive from the Subclaims.

Other features and advantages of the present invention will be apparent from the following description taken in conjunction with the accompanying drawings, in which like reference characters designate the same or similar parts throughout the figures thereof.

## BRIEF DESCRIPTION OF THE DRAWINGS

Fig. 1 is a side view, partially shown in section, illustrating an embodiment of an intra-aortic balloon apparatus according to the present invention;

Fig. 2 is an enlarged sectional view illustrating a pressure transducer included in the apparatus of Fig. 1; and

Fig. 3 is a side view, partially shown in section, illustrating another embodiment of an intra-aortic balloon apparatus according to the present invention.

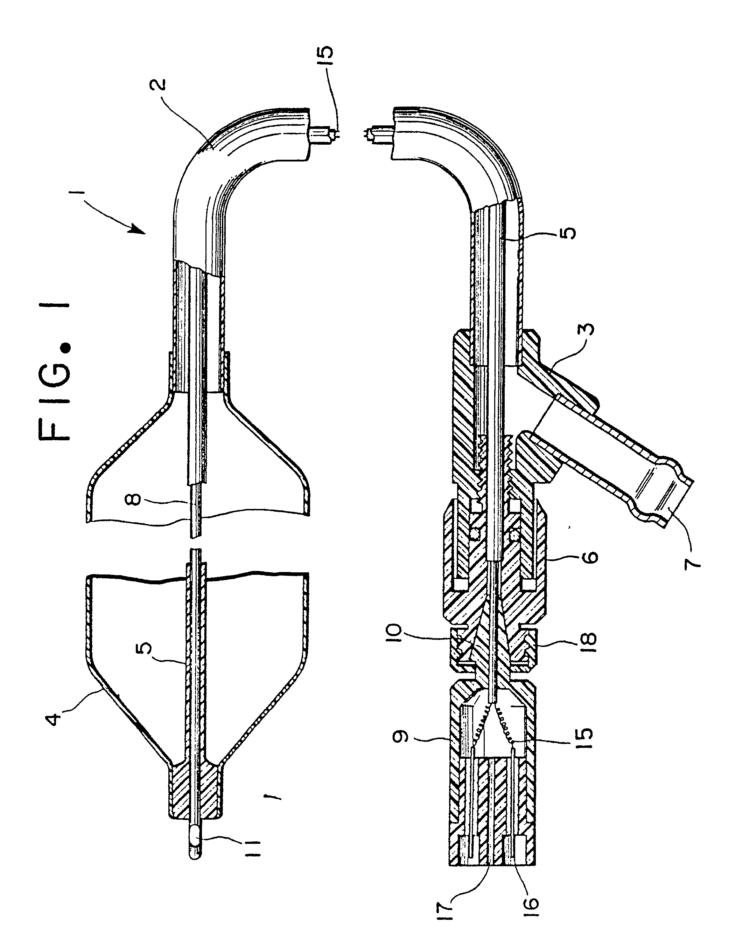

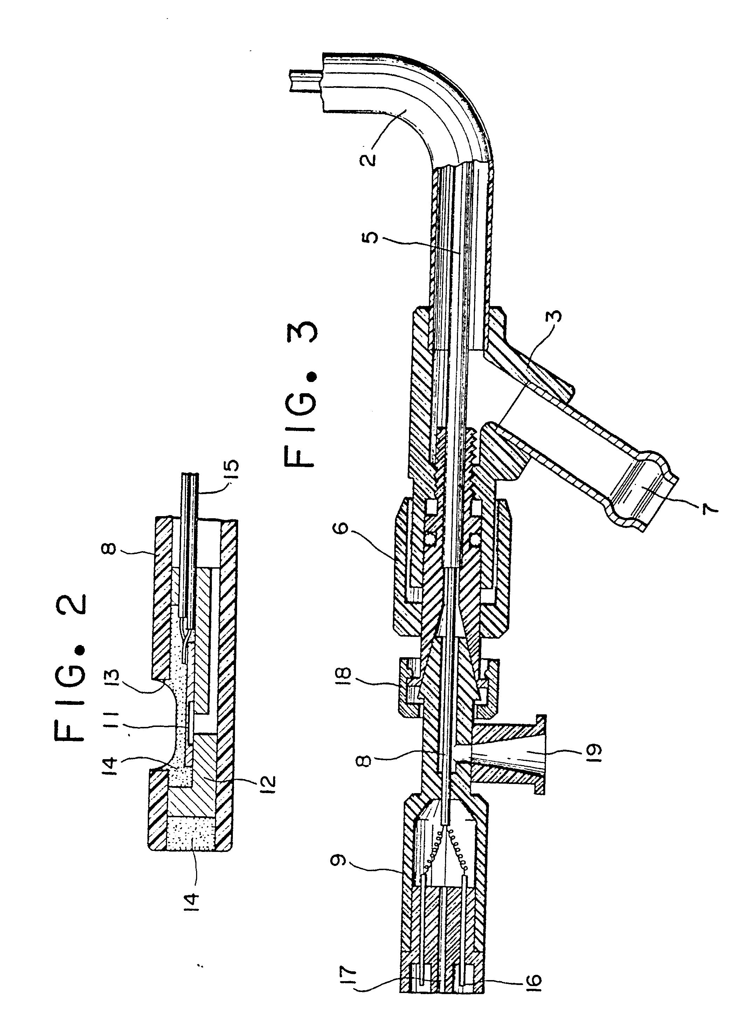

An embodiment of an intra-aortic balloon pump apparatus will now be described with reference to Figs. 1 and 2. The apparatus, shown generally at numeral 1, includes a tubular first catheter 2 having a proximate end joined to a Y-shaped first connector 3 and a distal end supporting the proximate end of a balloon 4 air-tightly. A central tubular member 5 extends through the interior of the first catheter 2 and has a distal end of comparatively larger diameter projecting from the distal end of the first catheter 2 and air-tightly supporting the distal end of the balloon 4.

The central tubular member 5 has a proximate end fixedly secured to a rotary member 6 having a portion in threaded engagement with the connector 3. Turning the rotary member 6 with respect to the connector 3 rotates the central tubular member 5 so that the balloon 4 may be wrapped around the central tubular member 5 by rotating the same in one direction and unwrapped from the central tubular member 5 by rotating same in the opposite direction. The connector 3 has a gas supply port 7 from which a fluid such as helium gas is introduced from an external source, not shown. The gas is fed from the connector 3 into the balloon 4 through a passageway defined between the inner surface of the first catheter 2 and the outer surface of the central tubular member 5, whereby the balloon 4 is made to inflate. Withdrawing the gas from the balloon 4 through the same passageway causes the balloon 4 to deflate.

A tubular second catheter 8, the diameter of which is smaller than that of the first catheter 2, is capable of being passed through the passageway of the central tubular member 5 and has a distal end that projects from the distal end of the central tubular member 5. The second catheter 8 has a proximate end secured to a second connector 9. The second connector 9 has a tapered distal end portion 10 lockably fitted into a tapered bore formed inside the rotary member 6. A knob 18 is provided for releasably locking the second connector 9 to the rotary member 6.

Disposed on the distal end portion of the second catheter 8 is a pressure transducer 11, as shown in Fig. 2. The transducer 11 is adapted to sense aortic pressure and produce a signal indicative thereof. Specifically, the pressure transducer 11 is attached to a metal block 12 secured to the inner wall surface of the second catheter 8 so as to face a pressure detection opening 13 formed in a portion of the catheter wall. The tip of the second catheter 8 and the pressure detection opening 13 are filled with silicone material 14 for holding the metal block 12 and, hence, the pressure transducer 11, in place inside the distal end of the second catheter 8. The pressure transducer 11 has lead wires 15 which pass through the interior of the second catheter 8 and extend into the second connector 9, where they are electrically connected to terminals 16 provided inside the connector 9. The terminals 16 of the second connector 9 thus receive the aortic pressure signal from the pressure transducer 11. The second connector 9 is in turn connected to electronic circuitry, not shown, the function of which is described hereinbelow. The second connector 9 is formed to include an air passage 17 for communicating the external atmosphere with the underside of the pressure transducer 11, i.e., the side of the transducer 11 facing away front the pressure detection opening 13, so that this side of the transducer 11 is maintained at a constant pressure at all times. In a preferred embodiment, the second catheter 8 comprises a stainless steel tube, the outer diameter of which is not more than 0.8 mm.

To use the intra-aortic balloon pump apparatus 1 shown in Fig. 1, first a guide wire, not shown, is introduced into the aorta via the patient's femoral artery as by the Seldinger technique until the leading end of the guide wire reaches the aortic arch. Next, the other end of the guide wire is inserted into the distal end of the central tubular member 5, into which the second catheter 8 has not yet been introduced. The central tubular member 5, with the balloon 4 wrapped around it, is then advanced together with the first catheter 2 into the aorta while being guided along the guide wire. When these have reached a predetermined position inside the aorta, the guide wire is withdrawn from the patient's body through proximate end of the central tubular member 5.

Next the distal end of the second catheter 8 is inserted into the central tubular member 5 and advanced while manipulating the second connector 9 to which the second catheter 8 is affixed, until the pressure transducer 11 at the distal end of the second catheter 8 is made to project into the aorta from the distal end of the central tubular member 5. The tapered distal end portion 10 of the second connector 9 is then brought into abutting contact with the rotary member 6 and the knob 18 is turned to lock the second connector 9 and the rotary member 6 together. Now the rotary member 6 is turned to unwrap the balloon 4 from the central tubular member 5, thereby setting the balloon 4 at the predetermined position in the aorta. The connector 9 is connected to the aforementioned electronic circuit, which thus receives the aortic pressure signal from the pressure transducer 11 at the distal end of the second catheter 8 via the lead wires 15 and terminals 16 and calculates the appropriate counterpulsation timing in accordance with the aortic pressure signal.

The intra-aortic balloon apparatus 1 is capable of being adapted to function without requiring the step of inserting the second catheter 8. In such an arrangement, the tapered distal end portion 10 of the second connector 9 may be replaced by a tapered plug engaged with the rotary member 6.

Another embodiment is illustrated in Fig. 3. Here a portion of the second connector 9 is provided with a port 19 communicating with the aorta through a passageway defined between the central tubular member 5 and the second catheter 8. The port 19 is useful for feeding a medical fluid into the aorta during the operation of the intra-aortic balloon pump apparatus 1. The structure is the same as that shown in Fig. 1 in other respects and identical portions are designated by like reference characters and need not be described again.

It should be clear from the above description that the passageway defined inside the central tubular member 5 has multiple uses, it is used for introducing the balloon pump into the patient's aorta along the guide wire inserted beforehand, and for the placement of the second catheter 8 having the pressure transducer 11 provided thereon close to the patient's heart. This makes it possible to accurately measure arterial pressure and, hence, to achieve counterpulsation timing far more precise than that obtainable in the prior art.

## Claims

1. An intra-aortic balloon apparatus comprising
   a first connector (3) having a gas supply port (7),
   a tubular first catheter (2) having a proximate end and a distal end, the proximate end being fixedly secured to said first connector (3),
   a balloon (4) having a proximate end and a distal end, the proximate end being fixedly secured to the distal end of said first catheter (2),
   a central tubular member (5) inserted in said first catheter (2) and defining therein a passageway extending the length thereof and having a distal end projecting from the distal end of said first catheter (2) and a proximate end extending through said first connector (3), the distal end of said balloon (4) being fixedly secured to the distal end of said central tubular member (5),
   said balloon (4), said central tubular member (5) and said first catheter (2) being insertable into the aorta of a patient,
   **characterized by**
   a second catheter (8) freely insertable into the passageway of said central tubular member (5) and having a distal end and a proximate end, the length between said distal and proximate ends thereof being longer than that of said central tubular member (5), the distal end of said second catheter (8) being provided with a pressure transducer (11),
   a second connector (9) having a proximate end to which the proximate end of said second catheter (8) can be fixedly secured for feeding a signal produced by said pressure transducer (11) to the outside of the apparatus,
   said second catheter (8) being insertable into said passageway in said central tubular member (5) with said pressure transducer (11) being projectable into the aorta from the distal end of said central tubular member (5).

2. The apparatus according to claim 1, further comprising a rotary member (6) fixedly secured to said proximate end of said central tubular member (5) and having a portion engaged with said second connector (9).

3. The apparatus according to claim 2, said portion of said rotary member (6) being formed with a tapered bore so as to be lockably fitted with a tapered distal end portion (10) of said second connector (9).

## Revendications

1. Dispositif à ballon intra-aortique comprenant
   un premier connecteur (3) comportant un orifice d'alimentation en gaz (7),
   un premier cathéter tubulaire (2) ayant une extrémité proche et une extrémité éloignée, l'extrémité proche étant liée de manière fixe audit premier connecteur (3),
   un ballon (4) ayant une extrémité proche et une extrémité éloignée, l'extrémité proche étant liée de façon fixe à l'extrémité éloignée dudit premier cathéter (2),
   un organe tubulaire central (5) inséré dans ledit premier cathéter (2) et définissant dans celui-ci un passage se prolongeant sur sa longueur et ayant une extrémité éloignée dépassant de l'extrémité éloignée dudit premier cathéter (2) et une extrémité proche s'étendant au travers dudit premier connecteur (3), l'extrémité éloignée dudit ballon (4) étant liée de façon fixe à l'extrémité éloignée dudit organe tubulaire central (5),
   ledit ballon (4), ledit organe tubulaire central (5) et ledit premier cathéter (2) pouvant être insérés dans l'aorte d'un patient,
   caractérisé par
   un second cathéter (8) pouvant être inséré librement dans le passage dudit organe tubulaire central (5) et ayant une extrémité éloignée et une extrémité proche, la longueur séparant lesdites extrémités éloignée et proche de celui-ci étant supérieure à celle dudit organe tubulaire central (5), l'extrémité éloignée dudit second cathéter (8) étant munie d'un transducteur de pression (11),
   un second connecteur (9) ayant une extrémité proche à laquelle l'extrémité proche dudit second cathéter (8) peut être liée de façon fixe pour fournir un signal produit par ledit transducteur de pression (11) à l'extérieur de l'appareil,
   ledit second cathéter (8) pouvant être inséré dans ledit passage dudit organe tubulaire central (5), ledit transducteur de pression (11) pouvant faire saillie à l'intérieur de l'aorte par rapport à l'extrémité éloignée dudit organe tubulaire central (5).

2. Dispositif selon la revendication 1, comprenant en outre un organe rotatif (6) lié de façon fixe à ladite extrémité proche dudit organe tubulaire centra (5) et ayant une partie s'engageant dans ledit second connecteur (9).

3. Appareil selon la revendication 2, ladite partie dudit organe rotatif (6) étant réalisée de manière à présenter un alésage biseauté pour pouvoir être ajustée de manière verrouillable dans une partie d'extrémité éloignée biseautée (10) dudit second connecteur (9).

## Ansprüche

1. Intraaortisches Ballongerät, das umfaßt ein erstes Anschlußteil (3) mit einem Gaszufuhrkanal (7),

einen rohrförmigen ersten Katheter (2) mit einem nächstliegenden Ende sowie einem fernliegenden Ende, wobei das nächstliegende Ende fest an dem genannten ersten Anschlußteil (3) angebracht ist,

einen Ballon (4) mit einem nächstliegenden Ende sowie einem fernliegenden Ende, wobei das nächstliegende Ende fest am fernliegenden Ende des besagten ersten Katheters (2) angebracht ist,

ein zentrales, rohrförmiges Element (5), das in den besagten ersten Katheter (2) eingesetzt ist sowie darin einen über seine Länge sich erstreckenden Durchgang bestimmt und ein fernliegendes, vom fernliegenden Ende des besagten ersten Katheters (2) vorragendes sowie ein durch das genannte erste Anschlußteil (3) verlaufendes naheliegendes Ende hat, wobei das fernliegende Ende des erwähnten Ballons (4) fest am fernliegenden Ende des besagten rohrförmigen Elements (5) angebracht ist,

wobei der erwähnte Ballon (4), das besagte rohrförmige Element (5) und der genannte erste Katheter (2) in die Aorta eines Patienten einführbar sind, gekennzeichnet durch

einen zweiten, frei in den Durchgang des besagten zentralen, rohrförmigen Elements (5) einsetzbaren und ein fernliegendes Ende sowie ein nächstliegendes Ende aufweisenden Katheter (8), dessen Länge zwischen seinem fern liegenden sowie nächstliegenden Ende länger ist als diejenige des besagten zentralen, röhrförmigen Elements (5), wobei das fernliegende Ende des erwähnten zweiten Katheters (8) mit einem Druckwandler (11) versehen ist,

ein zweites Anschlußteil (9) mit einem nächstliegenden Ende, an welchem das nächstliegende Ende des erwähnten zweiten Katheters (8) fest angebracht werden kann, um ein von dem genannten Druckwandler (11) erzeugtes Signal zur Außenseite des Geräts zu führen,

wobei der erwähnte zweite Katheter (8) in den genannten Durchgang in dem besagten zentralen, rohrförmigen Element (5) mit dem erwähnten, in die Aorta vom fernliegenden Ende des besagten zentralen, rohrförmigen Elements (5) vorstehfähigen Druckwandler (11) einführbar ist.

2. Gerät nach Anspruch 1, das ferner ein fest an dem erwähnten nächstliegenden Ende des besagten zentralen, rohrförmigen Elements (5) angebrachtes und einen mit dem genannten zweiten Anschlußteil (9) in Eingriff befindlichen Abschnitt aufweisendes Drehorgan (6) umfaßt.

3. Gerät nach Anspruch 2, wobei der genannte Abschnitt des erwähnten Drehorgans (6) mit einer kegelförmigen Bohrung versehen ist, so daß er verriegelbar mit einem kegelförmigen, fernliegenden Endstück (10) des genannten zweiten Anschlußteils (9) zusammenzubauen ist.

FIG. 1

FIG. 2

FIG. 3